# EUROPEAN PATENT SPECIFICATION

(11) **EP 4 084 690 B1**
(45) Date of publication and mention of the grant of the patent: **07.10.2026**
(21) Application number: 20909909.2
(22) Date of filing: 26.08.2020
(51) Int. Cl.: A61B 5/145, A61M 5/14, G16H 80/00, A61B 5/1473, A61B 5/00, A61M 5/142, A61M 5/145, A61M 5/158, A61M 5/172

(54) **INTEGRATED CLOSED-LOOP ARTIFICIAL PANCREAS**
INTEGRIERTE KÜNSTLICHE BAUCHSPEICHELDRÜSE MIT GESCHLOSSENEM REGELKREIS
PANCRÉAS ARTIFICIEL À BOUCLE FERMÉE INTÉGRÉ

(30) Priority: 31.12.2019 WO PCT/CN2019/130440
(43) Date of publication of application: 09.11.2022
(73) Proprietor: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2020/111177
(87) International publication number: WO 2021/135292

(56) References cited:
- CN-A- 101 912 641
- CN-A- 102 711 898
- CN-A- 108 261 585
- CN-A- 108 261 585
- US-A1- 2005 272 640
- US-A1- 2008 112 853
- US-A1- 2012 277 667
- US-A1- 2018 117 250
- US-A1- 2018 126 068

## Description

### TECHNICAL FIELD

The present invention mainly relates to the field of medical instruments, in particular to an integrated closed-loop artificial pancreas.

### BACKGROUND

Diabetes is mainly a metabolic disease caused by abnormal human pancreatic function. Diabetes is a lifelong disease. At present, medical technology cannot cure diabetes. It can only control the occurrence and development of diabetes and its complications by stabilizing blood glucose. The normal human pancreas automatically monitors changes in the body's blood glucose levels and automatically secretes the required insulin. At present, the medical device for stabilizing blood glucose works by dynamically monitoring the blood glucose changes of the human body by a glucose sensor implanted in the subcutaneous tissue of the human body; and continuously accurately infusing insulin into the subcutaneous tissue of the human body through a medical tube implanted in the subcutaneous tissue of the human body.

At present, the detection device and the infusion device are connected to each other to form a closed-loop artificial pancreas with the processing of the program module. While the program module is calculating the insulin infusion dose, total daily dose (TDD) is an important parameter with many determinants, such as physical conditions, physiological conditions, etc.

However, the device in prior art requires separately inserting glucose sensor and infusion tube under the human skin. Even though there are some devices that can integrate the sensor probe and the infusion tube into one device, the sensor and tube still need to be separately inserted at different positions, increasing the risk of infection. And at the same time, the device in prior art needs to be manually input the physical conditions instead of automatically detecting, and the TDD value cannot be accurately obtained, resulting in inaccurate current insulin infusion dose and worsening user experience. Exemplary devices are disclosed in US 2012/277667 and CN 108 261 585.

Therefore, there is a need in the prior art for an integrated closed-loop artificial pancreas that can perform both detection and infusion at the same time and automatically detect the physical condition and accurately calculate the current infusion dose.

### BRIEF SUMMARY OF THE INVENTION

Embodiments of the present invention disclose an integrated closed-loop artificial pancreas in which multiple electrodes are disposed on an infusion tube also acted as an infusion channel. It takes only one insertion to perform both glucose detection and insulin infusion, thus reducing the risk of infection. At the same time, the artificial pancreas can automatically detect the physical condition and accurately calculate the current infusion dose.

The invention discloses an integrated closed-loop artificial pancreas, comprising: a detection module, configured to detect blood glucose, includes at least two electrodes; a program module, connected to the detection module, is configured to obtain the insulin dose infused per day by users, and is also imported into the total daily dose algorithm and the current insulin infusion algorithm, wherein, according to the insulin dose infused per day by users, the total daily dose algorithm is used to calculate the total daily dose; according to the blood glucose detected, the insulin dose infused per day by users or total daily dose, the current insulin infusion algorithm is used to calculate the current insulin infusion dose; and an infusion module, connected to the program module, includes an infusion tube which is used as the insulin infusion channel, the detecting electrodes are provided on/in the wall of the infusion tube, and the infusion module can infuse insulin required according to the data of the current insulin infusion dose.

According to one aspect of this invention, the electrodes are located on the outer surface of the tube wall or inside the tube wall.

According to one aspect of this invention, the electrodes are located on the subcutaneous part of the outer surface of the tube wall, and the outer surface of the tube wall is further provided with electrode leads electrically connected to the electrodes.

According to one aspect of this invention, the infusion tube includes an inner layer tube and at least one outer layer tube, and the outer layer tubes are disposed outside the inner layer tube, and the inner layer tube is used for insulin infusion.

According to one aspect of this invention, at least one electrode is provided between the outer wall of the inner layer tube and the outermost tube.

According to one aspect of this invention, the electrode located on the outer wall surface of the inner layer tube is entirely exposed in the subcutaneous tissue fluid, or covered in whole or in part by the outer layer tubes.

According to one aspect of this invention, when the electrode located on the outer wall surface of the inner layer tube is covered in whole or in part by the outer layer tubes, the material of the outer layer tube walls is permeable membrane or a semi-permeable membrane.

According to one aspect of this invention, the electrodes include at least one working electrode and at least one auxiliary electrode.

According to one aspect of this invention, a plurality of electrodes form one or more electrode combinations, each electrode combination comprising working electrode and auxiliary electrode, the detection module choosing one or more electrode combinations to detect glucose data in body fluid.

According to one aspect of the present invention, the program module includes a manual input interface or an automatic detection sub-module, and the method for the program module to obtain the insulin dose infused per day by users includes: the insulin dose infused per day by users is manually input into the program module through the manual input interface; or the insulin dose infused per day by users is automatically detected, stored and calculated by the automatic detection sub-module.

According to one aspect of the present invention, the insulin dose infused per day by users includes the total amount of daily infusion dose data, or the bolus and basal data infused in different time periods, or the temporary basal data and the correction bolus data, or the infusion data after different events.

According to one aspect of the present invention, the total daily dose is obtained by calculating the total amount of daily infusion dose data in the previous two or more days according to the total daily dose algorithm, and the total daily dose is the average or median of the insulin dose infused per day by users, and the total daily dose is one variable factor of the current insulin infusion algorithm.

According to one aspect of the present invention, the variable factors of the total daily dose algorithm include one or more of the user's physical activity status, physiological status, psychological status, and meal status.

According to one aspect of the present invention, the physiological status includes one or more factors of weight, gender, age, disease, and menstrual period.

According to one aspect of the present invention, the physical activity status includes general body stretching, exercise, or sleep, and the physical activity is one variable factor of the current insulin infusion algorithm.

According to one aspect of the present invention, it further comprises a motion sensor, which is provided in the detection module, the program module or the infusion module, is used to automatically sense the user's physical activity status.

According to one aspect of the present invention, the motion sensor includes a three-axis acceleration sensor or a gyroscope.

According to one aspect of the present invention, the detection module, the program module and the infusion module are connected together configured to form a single structure which is attached on only one position on the skin.

Compared with the prior arts, the technical solution of the present invention has the following advantages:
In the integrated closed-loop artificial pancreas disclosed herein, at least two detecting electrodes are provided on/in the wall of the infusion tube. The infusion tube performs glucose detection and insulin infusion at the same time. Once the puncture is performed at one position, the glucose detection and the insulin infusion can be completed simultaneously, reducing the risk of the user's infection. Secondly, the program module is imported into the total daily dose algorithm and the current insulin infusion algorithm, wherein, according to the blood glucose detected, insulin dose infused per day by users or total daily dose, the current insulin infusion algorithm is used to calculate the current insulin infusion dose. The program module is imported into the total daily dose algorithm and the current insulin infusion algorithm. Using the detection data, the insulin dose infused per day by users and the total daily dose alone or in combination makes the current insulin infusion dose more accurate.

Furthermore, when the electrode located on the outer wall surface of the inner layer tube is covered in whole or in part by the outer layer tubes, the material of the outer layer tubes wall is permeable membrane or a semi-permeable membrane. The tube wall material is selected from a permeable membrane or a semi-permeable membrane to ensure the required analyte passes through the tube wall to the electrode surface. It can improve the flexibility of electrode position design without affecting the detection.

Furthermore, a plurality of electrodes constitute one or more electrode combinations, each electrode combination includes working electrode and auxiliary electrode, and the detection module selects one or more electrode combinations to detect the glucose data. On the one hand, when a combination of electrodes fails to detect, the detection module can select other electrode combinations for detection according to the situation to ensure the detection process of the body fluid signal is uninterrupted. On the other hand, the detection module can select multiple electrode combinations to work at the same time, performing statistical analysis on multiple sets of data of the same parameter at the same time, improving the detection accuracy of the glucose data, thus, making the program module issue a more accurate infusion signal.

Furthermore, the program module includes a manual input interface or an automatic detection sub-module, and the method for the program module to obtain the insulin dose infused per day by users includes: the insulin dose infused per day by users is manually input into the program module through the manual input interface; or the insulin dose infused per day by users is automatically detected, stored and calculated by the automatic detection sub-module. The manual input interface or the automatic detection sub-module can be used alone or a combined, which enhances the flexibility using the device. Secondly, with the manual input interface and the automatic detection sub-module used in combination, the data automatically detected and manually input can be combined and compared to make the program module adjust the algorithm in real time, helping to make the calculation result more accurate.

Furthermore, the physical activity status includes general body stretching, exercise or sleep. The artificial pancreas can distinguish normal activities, exercise and sleep, making the artificial pancreas more refined to control blood glucose level.

Furthermore, the motion sensor is provided in the detection module, the program module or the infusion module. The motion sensor provided in the artificial pancreas, not disposed in other structure, can improve the integration of the artificial pancreas as much as possible, reduce the size of the device, and enhance the user experience.

Furthermore, the motion sensor includes a three-axis acceleration sensor or a gyroscope. The three-axis acceleration sensor or gyroscope can sense the body's activity intensity, activity mode or body posture accurately, ultimately improving the accuracy of the calculation result of the infusion dose.

Furthermore, the detection module, the program module and the infusion module are connected together configured to form a single structure which is attached on only one position on the skin. If the three modules are connected as a whole and attached in the only one position, the number of the device on the user skin will be reduced, thereby reducing the interference of more attached devices on user activities. At the same time, it also effectively solves the problem of the poor wireless communication between separating devices, further enhancing the user experience.

### BRIEF DESCRIPTION OF THE DRAWINGS

FIG.1 is a schematic view of the module relationship of the integrated closed-loop artificial pancreas according to one embodiment of the present invention.
FIG.2 is a schematic view of an infusion tube of an integrated closed-loop artificial pancreas according to an embodiment of the present invention.
FIG.3a - FIG.3b are partial longitudinal views of an infusion tube including two electrodes according to one embodiment of the present invention.
FIG.4a - FIG.4c are partial longitudinal views of an infusion tube and the two electrodes according to another embodiment of the present invention.
FIG.5 is a partial longitudinal view of an infusion tube provided with three electrodes according to still another embodiment of the present invention.
FIG.6 is a partial longitudinal view of an infusion tube including an inner layer tube and one outer layer tube according to still another embodiment of the present invention.

### DETAILED DESCRIPTION

As described above, in the prior art device, the detection and the infusion are performed separately to control the glucose level in the body fluid, and it is necessary to puncture at multiple positions on the skin, thereby increasing the pain of the user and increasing the risk of infection. And at the same time, the device in prior art needs to be manually input the physical conditions instead of automatically detecting, and the TDD value cannot be accurately obtained, resulting in inaccurate current insulin infusion dose, and worsening user experience.

The study found that the cause of the above problems is that the sensor detection device and the insulin medical device are two independent units. Or even if the two are designed into a single structure, multiple puncture positions are still required on the body surface. And device in the prior art has insufficient algorithms, with single calculation method, for calculating the total daily dose.

In order to solve this problem, the present invention provides an integrated closed-loop artificial pancreas, and the infusion tube is used for detecting glucose data and an insulin infusion channel. And it can perform detection and infusion with only one puncture. And it can automatically detect the physical condition of the user and accurately calculate the TDD value and the current insulin infusion dose, enhancing user experience.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other structures.

The following description of the exemplary embodiments is merely illustrative, and is not intended to be in any way limiting the invention and its application or use. The techniques, methods and devices that are known to those of ordinary skill in the art may not be discussed in detail, but such techniques, methods and devices should be considered as part of the specification.

It should be noted that similar reference numerals and letters indicate similar items in the following figures. Therefore, once an item is defined or illustrated in a drawing, it will not be discussed further in the following description of the drawings.

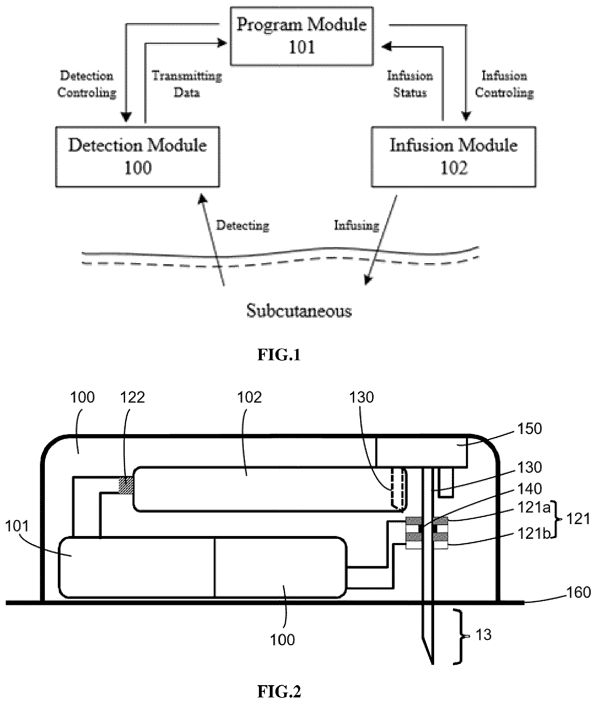

FIG.1 is a schematic view of the module relationship of the integrated closed-loop artificial pancreas according to the embodiment of the present invention.

The integrated closed-loop artificial pancreas disclosed in the embodiment of the present invention mainly includes a detection module 100, a program module 101, and an infusion module 102.

The detection module 100 is used to continuously detect the user's real-time blood glucose (BG) level. Generally, the detection module 100 is a Continuous Glucose Monitoring (CGM) for detecting real-time BG, monitoring BG changes, and also sending them to the program module 101.

The program module 101 is used to control the detection module 100 and the infusion module 102. Therefore, the program module 101 is connected to the detection module 100 and the infusion module 102, respectively. Here, the connection refers to a conventional electrical connection or a wireless connection.

The infusion module 102 includes the essential mechanical structures used to infuse insulin and controlled by the program module 101, which will be described in detail below. According to the current insulin infusion dose calculated by the program module 101, the infusion module 102 injects the currently insulin dose required into the user's body. At the same time, the real-time infusion status of the infusion module 102 can also be fed back to the program module 101.

The embodiment of the present invention does not limit the specific positions and connection relationships of the detection module 100, the program module 101 and the infusion module 102, as long as the aforementioned functional conditions can be satisfied.

As in an embodiment of the present invention, the three are electrically connected to form a single structure. Therefore, the three modules can be attached together on only one position of the user's skin. If the three modules are connected as a whole and attached in the only one position, the number of the device on the user skin will be reduced, thereby reducing the interference of more attached devices on user activities. At the same time, it also effectively solves the problem of the poor wireless communication between separating devices, further enhancing the user experience.

Here, it should be noted that the program module 101 of the embodiment of the present invention may also include multiple sub-modules. According to the functions of the sub-modules, different sub-modules can be respectively assembled in different part, which is not specific limitation herein, as long as the control conditions of the program module 101 can be satisfied.

In the embodiment of the present invention, the program module 101 is also used to obtain data including the insulin dose infused per day by users. Generally, for artificial pancreas, the current insulin dose required is closely related to the insulin dose infused per day by users in history. Preferably, in the embodiment of the present invention, the insulin dose infused per day by users includes the total amount of daily infusion dose data (d), or the bolus and basal data infused in different time periods, or the temporary basal data and the correction bolus data, or the infusion data after different events.

The program module 101 includes a manual input interface (not shown) or an automatic detection sub-module (not shown). By using the manual input interface or the automatic detection sub-module alone, or using the two combination, the program module 101 can obtain the user's physical condition data. This alone or combination using of these two modules enhances the flexibility in using the device.

For example, in an embodiment of the present invention, with the manual input interface, users can manually input the insulin dose infused per day by users into the program module 101 according to the clinical guidance. In another embodiment of the present invention, the program module 101 has already stored and recorded the user's previous insulin infusion data. With the automatic detection sub-module, the program module 101 can automatically obtain and calculate the insulin dose infused per day by users. Preferably, in the embodiment of the present invention, the user uses the manual input interface in combination with the automatic detection sub-module. At this time, the data automatically detected and the manually input can be combined and compared, making the program module 101 adjust the algorithm in real time for obtaining more accurate calculation outcome.

In other embodiments of the present invention, through the manual input interface, users can also input other information, such as meal information, exercise information, sleep information, and physical condition information into the program module 101, which is not specifically limited herein.

Generally, the purpose of using an artificial pancreas is to stabilize the BG level, that is, an appropriate dose of insulin needs to be infused into the user's body. However, the current insulin infusion dose is closely related to the total daily dose (TDD) which is an important factor influencing the current insulin infusion dose. Therefore, the program module 101 is imported into the total daily dose algorithm and the current insulin infusion algorithm, which are used to calculate the TDD and the current insulin infusion dose, respectively.

The current insulin infusion algorithm is used to calculate the current insulin infusion dose required. In the embodiment of the present invention, there are also many factors affecting the current insulin infusion dose, such as physical activity status, TDD, etc. Preferably, in the embodiment of the present invention, the TDD is one of the variable factors. Therefore, the more accurate the TDD or the more accurate the artificial pancreas sensing the user's activity status, the more accurate the current insulin infusion dose will be. And TDD can be obtained from calculating the aforementioned insulin dose infused per day by users according to the total daily dose algorithm. At the same time, the program module 101 can alone or in combination uses the detection data, the insulin dose infused per day by users and TDD data to calculate the current insulin infusion dose.

There are many factors that affect TDD, and some of them are related to the user's physical condition. Therefore, in the embodiment of the present invention, the variable factors of the total daily dose algorithm include one or more of the user's physical activity status, physiological status, psychological status, and meal status.

Here, the physiological status of the user includes one or more factors of weight, gender, age, disease condition, and menstrual period.

The user's psychological status includes emotional conditions such as anger, fear, depression, hyperactivity, and excitement.

The user's physical activity status includes general body stretching, exercise, or sleep. The artificial pancreas can distinguish normal activities, exercise and sleep, making the artificial pancreas more refined to control BG levels.

As mentioned above, TDD is obtained by the program module 101 by calculating the total amount of daily infusion dose data (d) in the previous two days or more according to the total daily dose algorithm. Preferably, in the embodiment of the present invention, TDD is obtained by the program module 101 by calculating the total amount of daily infusion dose data (d) in the previous seven days. Preferably, TDD is the average value of the total amount of daily infusion dose data (d).

In an embodiment of the present invention, if *d₇, d*₆, *... , d*₂, *d*₁ respectively represent the total amount of daily infusion dose data in the previous seventh day, the previous sixth day, ... , the day before yesterday, and yesterday, then: $TDD = \left({d}_{7}+{d}_{6}+…+{d}_{2}+{d}_{1}\right) / 7$ that is, TDD is the arithmetic average of the total amount of daily infusion dose data (d).

If the time is much closer to the today, the total amount of daily infusion dose data (d) is much closer to the actual TDD. Therefore, in another embodiment of the present invention, different *d*ₙ has different weights *γ*ₙ, such as the corresponding weights *γ*₇, *γ*₆, ... , *γ*₂, *γ*₁, then: $TDD = {γ}_{7} {d}_{7} + {γ}_{6} {d}_{6} + … + {γ}_{2} {d}_{2} + {γ}_{1} {d}_{1}$ that is, TDD is the weighted average of the total amount of insulin infused per day (*d*).

It should be noted that the embodiment of the present invention does not limit the statistical method of *d*ₙ. In yet another embodiment of the present invention, the TDD value can be determined by the median of the total amount of daily infusion dose data (d) in the previous seven days. In another embodiment of the present invention, the maximum value and minimum value of *d*ₙ may be eliminated firstly, and then the averaging process is performed. Another embodiment of the present invention introduces variance or standard deviation method with discarding points with larger errors firstly and then performing averaging processing. In other embodiments of the present invention, a method of combining weighted average with a sliding data frame may also be used to make the calculation result of TDD more accurate.

Here, it should be noted that the sliding data frame refers to select the data, like from previous five consecutive days, as a data frame for statistics. And according to the passage of time, the data frame as a whole moves backward for several days, but still keeps including data of another previous five consecutive days. For the specific statistical method of the data in the sliding data frame, please refer to the foresaid, which will not be repeated herein.

As mentioned above, both **TDD** and the current insulin infusion dose are affected by physical activities. Therefore, the integrated closed-loop artificial pancreas also includes a motion sensor (not shown) which is used to sense the user's physical activity. And the program module 101 can receive physical activity status information. The motion sensor can automatically and accurately sense the physical activity status of the user which will be sent to the program module 101, making the calculation result of the **TDD** or the current insulin infusion dose much more accurate, and enhancing the user experience. At the same time, providing the motion sensor in the module of the artificial pancreas can improve the integration of the artificial pancreas as much as possible, reduce the device size, and enhance the user experience.

The motion sensor is provided in the detection module 100, the program module 101 or the infusion module 102. Preferably, in the embodiment of the present invention, the motion sensor is provided in the program module 101.

It should be noted that the embodiment of the present invention does not limit the number of motion sensors and the installation positions of these multiple motion sensors, as long as the conditions for the motion sensor to sense the user's activity status can be satisfied.

The motion sensor includes a three-axis acceleration sensor or a gyroscope. The three-axis acceleration sensor or gyroscope can more accurately sense the body's activity intensity, activity mode or body posture, which ultimately makes the calculation result of the infusion more accurate. Preferably, in the embodiment of the present invention, the motion sensor is the combination of a three-axis acceleration sensor and a gyroscope.

FIG.2 is a view of an artificial pancreas according to an embodiment of the present invention, and the artificial pancreas is an integral structure.

In the embodiment of the present invention, the artificial pancreas includes an input end 121 and an output end 122. The input end 121 is used for receiving glucose data signal while the output end 122 is for transmitting the infusion instruction to the infusion module. Therefore, the input end 121, the output end 122 are connected to the detection module 100, the program module 101, respectively. The input end 121 includes electrically connective regions 121a and 121b. When in operation, the electrically connective region is electrically connected to the electrode or electrode lead to receive the glucose signal. In other embodiments of the invention, the input end 121 may also include more electrically connective regions depending on the number of electrodes.

During the use of the integrated closed-loop artificial pancreas of the embodiment of the present invention, the infusion tube 130 can slide relative to the input end 121, while the input end 121 is provided as an elastic member. The elastic member is to ensure an interference fit between the infusion tube 130 and the input end 121 to avoid poor electrical contact. The elastic member includes: conductive rubber strip, oriented conductive silica gel, conductive ring, conductive ball, etc. When the number of electrodes is relatively large, the electrically connective regions are relatively dense. In this case, according to different structural designs, the elastic members may be one or more combinations of the above. Here, the infusion tube 130 includes infusion cannula or infusion needle. Or the infusion tube 130 is constituted by infusion cannula or infusion needle.

In an embodiment of the invention, when the infusion tube 130 is installed to the working position, the mounting unit 150 is pressed into the artificial pancreas with the top portion integral with the artificial pancreas housing, as shown in FIG.2.

In other embodiments of the invention, the infusion tube 130 further includes an electrical contact region 140 coupled to the input end 121. As shown in FIG.2, one end of the infusion tube 130 is inserted subcutaneously (indicated by the solid line portion of the infusion tube in FIG.2) and the other end (illustrated by the dotted portion of the infusion tube in FIG.2) is connected with the outlet of the infusion module 102, thereby establishing a flow path for the insulin from the infusion module 102 to the body tissue fluid. At the same time, the electrical contact region 140 contacts with the electrically connective region of the input end 121, enabling electrical connection between the detection module 100 and the electrical contact region 140.

In an embodiment of the invention, a medical tape 160 for attaching the artificial pancreas to the skin surface is used to paste the program module 101, the infusion module 102, the detection module 100 and the infusion tube 130 as a whole on the skin.

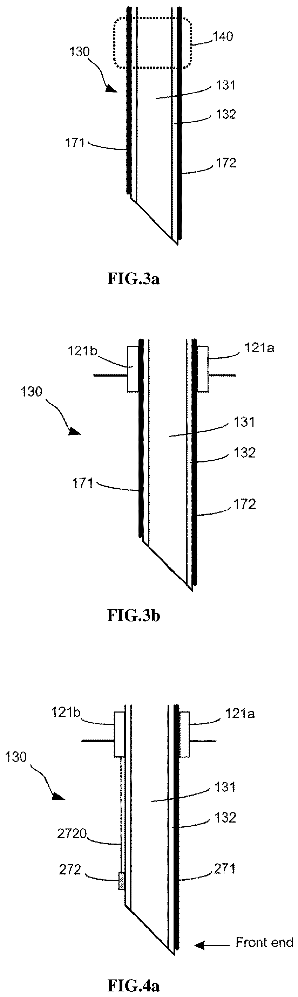

FIG.3a - FIG.3b are partial longitudinal views of the infusion tube 130 including two electrodes.

In the embodiment of the invention, the artificial pancreas includes at least two detecting electrodes that are disposed on the wall of the infusion tube 130, as shown in FIG.3a. The different electrodes are electrically connected to the electrically connective regions at the position of the dotted frame 140. The cavity 131 of the infusion tube 130 is used for insulin infusion.

In the embodiment of the present invention, the electrodes, such as electrode 171 and electrode 172, are plated on the outer surface of the tube wall of the infusion tube 130. The electrode 171 and the electrode 172, electrically insulated from each other, are directly electrically connected to the electrically connective regions 121a and 121b of the input end, respectively, which allows electrical signals of the glucose data to be transmitted to detection module 100, as shown in FIG.3b. Once the puncture is performed at one position, the glucose detection and the insulin infusion can be completed simultaneously, reducing the risk of the user's infection.

It should be noted that, in the embodiment of the present invention, a part of the electrode 171 or the electrode 172 is located in the subcutaneous tissue fluid, while another part is located above the skin, so that electrical signals can be transmitted on the electrode. The corresponding electrode arrangements in the other embodiments below have the same function and will not be described in detail later.

In the embodiment of the present invention, the artificial pancreas has only two electrodes, the electrode 171 is a working electrode while the electrode 172 is an auxiliary electrode. In another embodiment of the invention, the electrode 171 is an auxiliary electrode while the electrode 172 is a working electrode. The auxiliary electrode is a counter electrode.

In other embodiments of the present invention, more electrodes, which are electrically insulated from each other, may be provided on the surface of the infusion tube 130.

FIG.4a - FIG.4c are partial longitudinal views of an infusion tube 130 in accordance with another embodiment of the present invention.

It should be noted that the electrodes or electrode leads in all embodiments of the present invention are coated or plated on the infusion tube 130, but for ease of marking and description, the electrode leads or electrodes and the infusion tube will be depicted separately in the FIG.s. The following related structural views are the same as those here, which will not be described in detail below.

In this embodiment, the tube wall 132 of the infusion tube 130 provides with the electrode 271 and the electrode 272. And the electrode 271 is directly electrically connected to the electrically connective regions 121a, such as the electrode 171 in FIG.3a. The electrode 272 is disposed at the front end of the infusion tube 130. And an electrode lead 2720 is used to electrically connect to the electrode 272 and the electrically connective regions 121b. The electrode 272 is located on the subcutaneous part of the outer surface of the tube wall 132, while a part of the electrode 272 is located in the subcutaneous tissue fluid and another part is located above the skin. At this time, the electrode 272 is indirectly electrically connected to the electrically connective regions 121b, sending parameter information to the detection module 100.

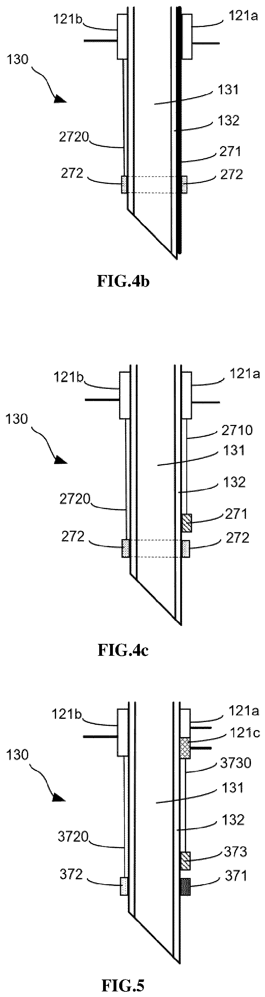

The embodiment of the present invention does not specifically limit the shape of the electrode 272. If the electrode 272 may be ring-shaped, the electrode 272 surrounds the front end of the infusion tube 130, as shown in FIG.4b. At this time, an insulation layer is provided between the electrode 272 and the electrode 271. As shown in FIG.4c, in yet another embodiment of the present invention, the electrode 271 and the electrode 272 are both provided at the front end of the infusion tube 130, that is, on the subcutaneous part of the outer surface of the tube wall. The outer surface of the tube wall 132 is also provided with an electrode lead 2710 and an electrode lead 2720 that are electrically connected to the electrode 271 and the electrode 272, respectively. The electrically connective regions 121a and 121b at the input end are electrically connected to the electrode lead 2710 and the electrode lead 2720, respectively. Therefore, the electrode 271 and the electrode 272 are indirectly electrically connected to the input end, transmitting the body fluid parameter signal to the detection module 100. During detection, both the electrode 271 and the electrode 272 are located in the subcutaneous tissue fluid.

As shown in FIG.4c, the electrode 272 is arranged in a ring shape surrounding a part of the outer surface of the tube wall 132. The electrode 271 and the electrode 272 may have other shapes, which is not specifically limited herein.

FIG.5 is a partial longitudinal view of an infusion tube 130 provided with three electrodes in accordance with yet another embodiment of the present invention.

In the embodiment of the present invention, three electrodes are disposed on the infusion tube 130: the electrode 371, 372 and 373 which are all disposed on the outer surface of the tube wall 132. Similarly, the surface of the tube wall 132 is also provided with electrode leads 3720 and 3730 which are electrically connected to the electrode 372 and the electrode 373, respectively. Similarly, the outer surface of the tube wall 132 is also provided with an electrode lead electrically connected to the electrode 371, but it is not shown in order to simplify the marking. The electrode lead of the electrode 371, electrode lead 3720 and electrode lead 3730 are electrically connected to the electrically connective regions 121a, 121b, and 121c of the input end, respectively, connecting the input end to each electrode. The shapes of the three electrodes can be various, and there is no specific limitation herein.

In the embodiment of the present invention, in order to simplify the design of the electrically connective region, the elastic member at the input end is an oriented conductive silica gel or a conductive ring. By doping different elements in the silica gel, it is possible to achieve directional conduction, such as horizontal conduction or vertical conductivity. Thus, even if 121a and 121c are adjacent to each other, the two can still be electrically insulated from each other. The electrically connective region 121b may be a conductive rubber strip or a conductive ball or the like, which is not specifically limited herein.

In the embodiment of the present invention, the electrode 371 is a working electrode, and the electrode 372 and the electrode 373 are both auxiliary electrodes. At this time, the electrode 371 and the electrode 372 or the electrode 373 may constitute a different electrode combination, that is, the two electrode combinations share the electrode 371. The detection module 100 can select different electrode combinations to detect glucose data. After the electrode combination is formed, on the one hand, when a working electrode combination fails to detect, the detection module 100 can select other electrode combinations for detection according to the situation to ensure that the detection process of the body fluid signal is uninterrupted. On the other hand, the detection module 100 can select a plurality of electrode combinations to work simultaneously, perform statistical analysis on multiple sets of data of the same parameter at the same time, improve the accuracy of the glucose data, thereby making the program module 101 output a more accurate insulin infusion signal.

In another embodiment of the present invention, the electrode 371, electrode 372, and electrode 373 include an auxiliary electrode and two working electrodes, which can also be arbitrarily selected according to actual needs, which are not specifically limited herein.

As an embodiment of the present invention, the electrode 371 is a working electrode, the electrodes 372 and 373 are auxiliary electrodes which are used as a counter electrode and a reference electrode, respectively, thereby forming a three-electrode system. Similarly, the three electrodes can be arbitrarily selected according to actual needs, which are not specifically limited herein.

Also, in other embodiments of the invention, more electrodes may be provided. The system includes a plurality of working electrodes and a plurality of auxiliary electrodes. At this time, each electrode combination includes at least a working electrode and an auxiliary electrode, and thus a plurality of electrodes may constitute a plurality of electrode combinations. The detection module 100 may select one or more electrode combinations to detect glucose data, as desired.

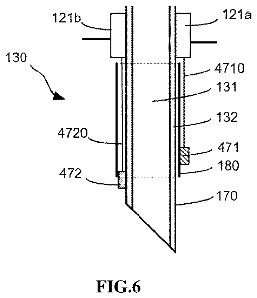

FIG.6 is a partial longitudinal view of an infusion tube 130 including an inner layer tube 170 and one outer layer tube 180 in accordance with yet another embodiment of the present invention.

The cavity 131 of the inner layer tube 170 is used as an insulin infusion channel. The tube wall of the infusion tube 130 includes the inner layer tube wall and the outer layer tube wall. The electrode 472 is disposed outside the tube wall of the inner layer tube 170, while the electrode 471 is provided on the outer surface of the wall of the outer layer tube 180. At this time, the electrode 472 is disposed in the wall of the infusion tube 130, that is, the electrode 472 is embedded between the outer layer tube 180 and the inner layer tube 170.

In the embodiment of the present invention, the electrode 472 may be partially covered by the outer layer tube 180 (as shown in FIG.6), or completely covered by the outer layer tube 180. The electrode 472 is electrically connected to the electrically connective region 121b through an electrode lead 4720, while the electrode 471 is electrically connected to the electrically connective region 121a through an electrode wire 4710. When the electrode 472 is partially or completely covered by the outer layer tube 180, the wall material of the outer layer tube 180 is a permeable membrane or a semi-permeable membrane. Such selection can facilitate the body fluid analyte to pass through the wall of the outer layer tube 180 and to be detected by the electrode, thereby improving the flexibility of electrode position design without affecting the detection.

In another embodiment of the present invention, the electrode 471 and the electrode 472 are both disposed in the wall of the infusion tube 130, that is, the electrode 471 and the electrode 472, which are completely covered by the outer layer tube 180, are both embedded between the inner layer tube 170 and the outer layer tube 180. At this time, the material of the outer layer tube 180 is as described above, which makes analytes detected by the electrode through the outer layer tube 180.

It should be noted that, in other embodiments of the present invention, more layers of outer layer tubes may be arranged outside the inner layer tube 170. And as described above, more electrodes can be provided on the infusion tube 130. According to actual needs, different electrodes can be arranged between different outer layer tubes. And at least one electrode is disposed between the wall of the inner layer tube and the outermost tube.

In addition to embedding electrodes into the wall of the infusion tube 130, some embodiments of the present invention can also reduce the length of the outer layer tube 180 in FIG.6, directly exposing the electrode 472 disposed on the outer surface of the inner layer tube 170 in tissue fluid.

In summary, the present invention discloses an integrated closed-loop artificial pancreas in which multiple electrodes are disposed on an infusion tube also acted as an infusion channel. It takes only one insertion to perform both glucose detection and insulin infusion, thus reducing the risk of infection. At the same time, the artificial pancreas can automatically detect the physical condition and accurately calculate the current infusion dose.

While the invention has been described in detail with reference to the specific embodiments of the present invention, it should be understood that it will be appreciated by those skilled in the art that the above embodiments may be modified without departing from the scope of the invention. The scope of the invention is defined by the appended claims.

## Claims

1. An integrated closed-loop artificial pancreas comprising:
a detection module (100), configured to continuously detect a real-time blood glucose level, includes at least two electrodes (171, 172, 271, 272, 371, 372, 373, 471, 472);
a program module (101), connected to the detection module (100); and an infusion module (102), connected to the program module (101), includes an infusion tube (130) which is used as an insulin infusion channel, wherein the electrodes (171, 172, 271, 272, 371, 372, 373, 471, 472) are provided on/in a tube wall (132) of the infusion tube (130); **characterised in that** the program module is configured to obtain an insulin dose infused per day by users, and import it into a total daily dose algorithm and a current insulin infusion algorithm, wherein,
according to the insulin dose infused per day by users, the total daily dose algorithm is used to calculate the total daily dose; and
according to a blood glucose detected, the insulin dose infused per day by users or the total daily dose, the current insulin infusion algorithm is used to calculate a current insulin infusion dose; wherein, the infusion module (102) is configured to
infuse insulin required according to a data of the current insulin infusion dose.

2. An integrated closed-loop artificial pancreas of claim 1, wherein:
the electrodes (171, 172, 271, 272, 371, 372, 373, 471, 472) are located on an outer surface of the tube wall (132) or inside the tube wall (132).

3. An integrated closed-loop artificial pancreas of claim 2, wherein:
the electrodes (271, 272) are located on a subcutaneous part of the outer surface of the tube wall (132), and the outer surface of the tube wall (132) is further provided with electrode leads (2710, 2720) electrically connected to the electrodes (271, 272).

4. An integrated closed-loop artificial pancreas of claim 2, wherein:
the infusion tube (130) includes an inner layer tube (170) and outer layer tubes (180), and the outer layer tubes are disposed outside the inner layer tube (170), and the inner layer tube (170) is used for insulin infusion.

5. An integrated closed-loop artificial pancreas of claim 4, wherein:
at least two electrodes (471, 472) are provided between the outer wall of the inner layer tube (170) and the outermost tube (180).

6. An integrated closed-loop artificial pancreas of claim 5, wherein:
the electrode located (472) on the outer wall surface of the inner layer tube (170) is entirely exposed in the subcutaneous tissue fluid, or covered in whole or in part by the outer layer tubes (180),
when the electrode located (472) on the outer wall surface of the inner layer tube (170) is covered in whole or in part by the outer layer tubes (180), a material of the outer layer tubes is a permeable membrane or a semi-permeable membrane.

7. An integrated closed-loop artificial pancreas of claim 1, wherein:
the electrodes (171, 172, 271, 272, 371, 372, 373, 471, 472) include at least one working electrode (371) and at least one auxiliary electrode (372, 373).

8. An integrated closed-loop artificial pancreas of claim 7, wherein:
the electrodes (171, 172, 271, 272, 371, 372, 373, 471, 472) form one or more electrode combinations, each of the electrode combinations comprises the working electrode (371) and the auxiliary electrode (372, 373), the detection module (100) chooses the one or more electrode combinations to detect glucose data in body fluid.

9. An integrated closed-loop artificial pancreas of claim 1, **characterized in that**:
the program module (101) includes a manual input interface or an automatic detection sub-module, and the method for the program module (101) to obtain the insulin dose infused per day by users includes:
the insulin dose infused per day by users is manually input into the program module through the manual input interface; or
the insulin dose infused per day by users is automatically detected, stored and calculated by the automatic detection sub-module.

10. An integrated closed-loop artificial pancreas of claim 9, wherein:
the insulin dose infused per day by users includes a total amount of daily infusion dose data, or bolus and basal data infused in different time periods, or temporary basal data and correction bolus data, or the infusion data after different events.

11. An integrated closed-loop artificial pancreas of claim 10, wherein:
the total daily dose is obtained by calculating the total amount of the daily infusion dose data in the previous two or more days according to the total daily dose algorithm, and the total daily dose is an average or median of the insulin dose infused per day by users, and the total daily dose is one variable factor of the current insulin infusion algorithm.

12. An integrated closed-loop artificial pancreas of claim 10, wherein:
variable factors of the total daily dose algorithm include one or more of user's physical activity status, physiological status, psychological status, and meal status, and the physiological status includes one or more factors of weight, gender, age, disease, and menstrual period.

13. An integrated closed-loop artificial pancreas of claim 12, wherein:
the physical activity status includes general body stretching, exercise, or sleep, and the physical activity status is one of the variable factors of the total daily dose algorithm or the current insulin infusion algorithm.

14. An integrated closed-loop artificial pancreas of claim 1,
further comprising a motion sensor, wherein the motion sensor which is provided in the detection module, the program module or the infusion module, is used to automatically sense the user's physical activity status, the motion sensor includes a three-axis acceleration sensor or a gyroscope.

15. An integrated closed-loop artificial pancreas of claim 1, wherein:
the detection module (100), the program module (102) and the infusion module (102) are connected together configured to form a single structure which is attached on only one position on a skin.

## Patentansprüche

1. Ein integriertes künstliches Pankreas mit geschlossenem Regelkreis, umfassend:
ein Detektionsmodul (100), das dazu ausgelegt ist, kontinuierlich einen Echtzeit-Blutzuckerspiegel zu detektieren, und mindestens zwei Elektroden (171, 172, 271, 272, 371, 372, 373, 471, 472) umfasst;
ein Programmmodul (101), das mit dem Detektionsmodul (100) verbunden und dazu ausgelegt ist, eine von Benutzern pro Tag infundierte Insulindosis zu erhalten, und in einen Algorithmus für die Gesamttagesdosis und einen Algorithmus für die aktuelle Insulininfusion einzugeben, wobei
der Algorithmus für die Gesamttagesdosis dazu verwendet wird, anhand der von Benutzern pro Tag infundierten Insulindosis die Gesamttagesdosis zu berechnen;
der Algorithmus für die aktuelle Insulininfusion dazu verwendet wird, anhand eines detektierten Blutzuckers, der von Benutzern pro Tag infundierten Insulindosis oder der Gesamttagesdosis eine aktuelle Insulininfusionsdosis zu berechnen; und
ein Infusionsmodul (102), das mit dem Programmmodul (101) verbunden ist und einen Infusionsschlauch (130) umfasst, der als Insulininfusionskanal verwendet wird, wobei die Elektroden (171, 172, 271, 272, 371, 372, 373, 471, 472) auf/in einer Schlauchwand (132) des Infusionsschlauchs (130) vorgesehen sind und das Infusionsmodul (102) das gemäß den Daten der aktuellen Insulininfusionsdosis erforderliche Insulin infundiert.

2. Integriertes künstliches Pankreas mit geschlossenem Regelkreis nach Anspruch 1, wobei:
die Elektroden (171, 172, 271, 272, 371, 372, 373, 471, 472) auf einer Außenfläche der Schlauchwand (132) oder innerhalb der Schlauchwand (132) angeordnet sind.

3. Integriertes künstliches Pankreas mit geschlossenem Regelkreis nach Anspruch 2, wobei:
die Elektroden (271, 272) auf einem subkutanen Abschnitt der Außenfläche der Schlauchwand (132) angeordnet sind und die Außenfläche der Schlauchwand (132) ferner mit Elektrodenleitungen (2710, 2720) versehen ist, die elektrisch mit den Elektroden (271, 272) verbunden sind.

4. Integriertes künstliches Pankreas mit geschlossenem Regelkreis nach Anspruch 2, wobei:
der Infusionsschlauch (130) einen Innenschichtschlauch (170) und Außenschichtschläuche (180) umfasst, wobei die Außenschichtschläuche außerhalb des Innenschichtschlauchs (170) angeordnet sind und der Innenschichtschlauch (170) zur Insulininfusion verwendet wird.

5. Integriertes künstliches Pankreas mit geschlossenem Regelkreis nach Anspruch 4, wobei:
mindestens zwei Elektroden (471, 472) zwischen der Außenwand des Innenschichtschlauchs (170) und dem äußersten Schlauch (180) vorgesehen sind.

6. Integriertes künstliches Pankreas mit geschlossenem Regelkreis nach Anspruch 5, wobei:
die auf der Außenwandfläche des Innenschichtschlauchs (170) angeordnete Elektrode (472) vollständig in der subkutanen Gewebeflüssigkeit freiliegt oder vollständig oder teilweise von den Außenschichtschläuchen (180) bedeckt ist,
wenn die auf der Außenwandfläche des Innenschichtschlauchs (170) angeordnete Elektrode (472) vollständig oder teilweise von den Außenschichtschläuchen (180) bedeckt ist, ein Material der Außenschichtschläuche eine permeable Membran oder eine semipermeable Membran ist.

7. Integriertes künstliches Pankreas mit geschlossenem Regelkreis nach Anspruch 1, wobei:
die Elektroden (171, 172, 271, 272, 371, 372, 373, 471, 472) mindestens eine Arbeitselektrode (371) und mindestens eine Hilfselektrode (372, 373) umfassen.

8. Integriertes künstliches Pankreas mit geschlossenem Regelkreis nach Anspruch 7, wobei:
die Elektroden (171, 172, 271, 272, 371, 372, 373, 471, 472) eine oder mehrere Elektrodenkombinationen bilden, jede der Elektrodenkombinationen die Arbeitselektrode (371) und die Hilfselektrode (372, 373) umfasst und das Detektionsmodul (100) die eine oder die mehreren Elektrodenkombinationen auswählt, um Glukosedaten in Körperflüssigkeit zu detektieren.

9. Integriertes künstliches Pankreas mit geschlossenem Regelkreis nach Anspruch 1, **dadurch gekennzeichnet, dass**:
das Programmmodul (101) eine manuelle Eingabeschnittstelle oder ein automatisches Detektionsuntermodul umfasst und das Verfahren, mit dem das Programmmodul (101) die von Benutzern pro Tag infundierte Insulindosis erhält, umfasst, dass:
die von Benutzern pro Tag infundierte Insulindosis über die manuelle Eingabeschnittstelle manuell in das Programmmodul eingegeben wird; oder
die von Benutzern pro Tag infundierte Insulindosis durch das automatische Detektionsuntermodul automatisch detektiert, gespeichert und berechnet wird.

10. Integriertes künstliches Pankreas mit geschlossenem Regelkreis nach Anspruch 9, wobei:
die von Benutzern pro Tag infundierte Insulindosis eine Gesamtmenge täglicher Infusionsdosisdaten oder zu unterschiedlichen Zeiträumen infundierte Bolus- und Basaldaten oder temporäre Basaldaten und Korrekturbolusdaten oder Infusionsdaten nach unterschiedlichen Ereignissen umfasst.

11. Integriertes künstliches Pankreas mit geschlossenem Regelkreis nach Anspruch 10, wobei:
die Gesamttagesdosis erhalten wird, indem gemäß dem Algorithmus für die Gesamttagesdosis die Gesamtmenge der täglichen Infusionsdosisdaten der vorausgegangenen zwei oder mehr Tage berechnet wird, wobei die Gesamttagesdosis ein Mittelwert oder Median der von Benutzern pro Tag infundierten Insulindosis ist und die Gesamttagesdosis ein variabler Faktor des Algorithmus für die aktuelle Insulininfusion ist.

12. Integriertes künstliches Pankreas mit geschlossenem Regelkreis nach Anspruch 10, wobei:
variable Faktoren des Algorithmus für die Gesamttagesdosis einen oder mehrere der folgenden Zustände des Benutzers umfassen: einen körperlichen Aktivitätszustand, einen physiologischen Zustand, einen psychischen Zustand und einen Mahlzeitenzustand, und der physiologische Zustand einen oder mehrere Faktoren aus Gewicht, Geschlecht, Alter, Krankheit und Menstruationsperiode umfasst.

13. Integriertes künstliches Pankreas mit geschlossenem Regelkreis nach Anspruch 12, wobei:
der körperliche Aktivitätszustand allgemeines Dehnen des Körpers, körperliche Betätigung oder Schlaf umfasst und der körperliche Aktivitätszustand einer der variablen Faktoren des Algorithmus für die Gesamttagesdosis oder des Algorithmus für die aktuelle Insulininfusion ist.

14. Integriertes künstliches Pankreas mit geschlossenem Regelkreis nach Anspruch 1,
ferner umfassend einen Bewegungssensor, wobei der Bewegungssensor, der in dem Detektionsmodul, dem Programmmodul oder dem Infusionsmodul vorgesehen ist, dazu verwendet wird, den körperlichen Aktivitätszustand des Benutzers automatisch zu erfassen, und der Bewegungssensor einen Dreiachsen-Beschleunigungssensor oder ein Gyroskop umfasst.

15. Integriertes künstliches Pankreas mit geschlossenem Regelkreis nach Anspruch 1, wobei:
das Detektionsmodul (100), das Programmmodul (101) und das Infusionsmodul (102) miteinander verbunden und dazu ausgelegt sind, eine einzige Struktur zu bilden, die an nur einer Position auf der Haut angebracht ist.

## Revendications

1. Un pancréas artificiel intégré en boucle fermée, comprenant :
un module de détection (100), configuré pour détecter en continu un taux de glucose sanguin en temps réel, comprenant au moins deux électrodes (171, 172, 271, 272, 371, 372, 373, 471, 472) ;
un module de programme (101), connecté au module de détection (100), configuré pour obtenir une dose d'insuline perfusée quotidiennement par des utilisateurs, et pour l'introduire dans un algorithme de dose quotidienne totale et un algorithme de perfusion actuelle d'insuline, dans lequel
l'algorithme de dose quotidienne totale est utilisé pour calculer la dose quotidienne totale en fonction de la dose d'insuline perfusée quotidiennement par des utilisateurs ;
l'algorithme de perfusion actuelle d'insuline est utilisé pour calculer une dose actuelle de perfusion d'insuline en fonction d'un glucose sanguin détecté, de la dose d'insuline perfusée quotidiennement par des utilisateurs ou de la dose quotidienne totale ; et
un module de perfusion (102), connecté au module de programme (101), comprenant un tube de perfusion (130) utilisé comme canal de perfusion d'insuline, dans lequel les électrodes (171, 172, 271, 272, 371, 372, 373, 471, 472) sont disposées sur/dans une paroi (132) du tube de perfusion (130), et le module de perfusion (102) perfuse l'insuline requise conformément aux données de la dose actuelle de perfusion d'insuline.

2. Pancréas artificiel intégré en boucle fermée selon la revendication 1, dans lequel :
les électrodes (171, 172, 271, 272, 371, 372, 373, 471, 472) sont situées sur une surface extérieure de la paroi (132) ou à l'intérieur de la paroi (132).

3. Pancréas artificiel intégré en boucle fermée selon la revendication 2, dans lequel :
les électrodes (271, 272) sont situées sur une partie sous-cutanée de la surface extérieure de la paroi (132), et la surface extérieure de la paroi (132) est en outre pourvue de conducteurs d'électrode (2710, 2720) électriquement connectés aux électrodes (271, 272).

4. Pancréas artificiel intégré en boucle fermée selon la revendication 2, dans lequel :
le tube de perfusion (130) comprend un tube de couche interne (170) et des tubes de couche externe (180), les tubes de couche externe étant disposés à l'extérieur du tube de couche interne (170), et le tube de couche interne (170) étant utilisé pour la perfusion d'insuline.

5. Pancréas artificiel intégré en boucle fermée selon la revendication 4, dans lequel :
au moins deux électrodes (471, 472) sont disposées entre la paroi extérieure du tube de couche interne (170) et le tube le plus externe (180).

6. Pancréas artificiel intégré en boucle fermée selon la revendication 5, dans lequel :
l'électrode (472) située sur la surface extérieure de la paroi du tube de couche interne (170) est entièrement exposée au liquide tissulaire sous-cutané, ou est entièrement ou partiellement recouverte par les tubes de couche externe (180),
lorsque l'électrode (472) située sur la surface extérieure de la paroi du tube de couche interne (170) est entièrement ou partiellement recouverte par les tubes de couche externe (180), un matériau des tubes de couche externe est une membrane perméable ou une membrane semi-perméable.

7. Pancréas artificiel intégré en boucle fermée selon la revendication 1, dans lequel :
les électrodes (171, 172, 271, 272, 371, 372, 373, 471, 472) comprennent au moins une électrode de travail (371) et au moins une électrode auxiliaire (372, 373).

8. Pancréas artificiel intégré en boucle fermée selon la revendication 7, dans lequel :
les électrodes (171, 172, 271, 272, 371, 372, 373, 471, 472) forment une ou plusieurs combinaisons d'électrodes, chacune des combinaisons d'électrodes comprenant l'électrode de travail (371) et l'électrode auxiliaire (372, 373), le module de détection (100) sélectionnant ladite une ou lesdites plusieurs combinaisons d'électrodes pour détecter des données de glucose dans un liquide corporel.

9. Pancréas artificiel intégré en boucle fermée selon la revendication 1, **caractérisé en ce que** :
le module de programme (101) comprend une interface de saisie manuelle ou un sous-module de détection automatique, et le procédé permettant au module de programme (101) d'obtenir la dose d'insuline perfusée quotidiennement par des utilisateurs comprend :
la saisie manuelle, dans le module de programme par l'intermédiaire de l'interface de saisie manuelle, de la dose d'insuline perfusée quotidiennement par des utilisateurs ; ou
la détection, le stockage et le calcul automatiques, par le sous-module de détection automatique, de la dose d'insuline perfusée quotidiennement par des utilisateurs.

10. Pancréas artificiel intégré en boucle fermée selon la revendication 9, dans lequel :
la dose d'insuline perfusée quotidiennement par des utilisateurs comprend une quantité totale de données de dose de perfusion quotidienne, ou des données de bolus et de débit basal perfusées pendant différentes périodes, ou des données de débit basal temporaire et des données de bolus de correction, ou des données de perfusion après différents événements.

11. Pancréas artificiel intégré en boucle fermée selon la revendication 10, dans lequel :
la dose quotidienne totale est obtenue en calculant, selon l'algorithme de dose quotidienne totale, la quantité totale des données de dose de perfusion quotidienne des deux jours précédents ou davantage, la dose quotidienne totale étant une moyenne ou une médiane de la dose d'insuline perfusée quotidiennement par des utilisateurs, et la dose quotidienne totale étant un facteur variable de l'algorithme de perfusion actuelle d'insuline.

12. Pancréas artificiel intégré en boucle fermée selon la revendication 10, dans lequel :
les facteurs variables de l'algorithme de dose quotidienne totale comprennent un ou plusieurs parmi l'état d'activité physique, l'état physiologique, l'état psychologique et l'état alimentaire de l'utilisateur, et l'état physiologique comprend un ou plusieurs facteurs parmi le poids, le sexe, l'âge, la maladie et la période menstruelle.

13. Pancréas artificiel intégré en boucle fermée selon la revendication 12, dans lequel :
l'état d'activité physique comprend un étirement général du corps, un exercice physique ou le sommeil, et l'état d'activité physique est l'un des facteurs variables de l'algorithme de dose quotidienne totale ou de l'algorithme de perfusion actuelle d'insuline.

14. Pancréas artificiel intégré en boucle fermée selon la revendication 1,
comprenant en outre un capteur de mouvement, dans lequel le capteur de mouvement, qui est disposé dans le module de détection, le module de programme ou le module de perfusion, est utilisé pour détecter automatiquement l'état d'activité physique de l'utilisateur, le capteur de mouvement comprenant un capteur d'accélération triaxial ou un gyroscope.

15. Pancréas artificiel intégré en boucle fermée selon la revendication 1, dans lequel :
le module de détection (100), le module de programme (101) et le module de perfusion (102) sont connectés ensemble et configurés pour former une structure unique qui est fixée en une seule position sur la peau.
